# EUROPEAN PATENT APPLICATION

(11) **EP 4 846 992 A1**
(43) Date of publication of application: **02.12.2026**
(21) Application number: 25194789.1
(22) Date of filing: 08.08.2025
(51) Int. Cl.: A61F 13/493, A61F 13/64, A61F 13/49, A61F 13/496

(54) **ADJUSTABLE ABSORBENT ARTICLES**

(30) Priority: 30.05.2025 EP 25179990
(71) Applicant: Ontex BV, 9255 Buggenhout (BE); Ontex Group NV, 9320 Erembodegem (BE)
(72) Inventor: Van Sande, Nathalie, 9688 Maarkedal (BE)
(74) Representative: Cobbaert, Dries

(57) **Abstract**

A disposable absorbent article, preferably a disposable pant, comprises elastic front and back panels, an absorbent insert, and an adjustable waist-tape. The adjustable waist-tape includes a first releasable attachment zone and a first permanent attachment zone, enabling adjustment of the waist circumference. The elastic material in the front and back panels can be an elastic film or strands. The front and back panels are joined by side seams, forming a pant-like structure. The releasable attachment zone may extend across side seams for a secure fit. The adjustable waist-tape may comprise a second releasable attachment zone for increased adjustability and may include elastic material for better fit. Temporary sealing zones may be used for manufacturing. The adjustable waist-tape design enhances comfort and prevents leakage by allowing customized fit around the waist.

## Description

### TECHNICAL FIELD

The present disclosure relates to disposable absorbent garments designed for the absorption and retention of bodily fluids, with a particular focus on enhancing the fit and leakage prevention capabilities of such garments through the incorporation of adjustable waist features. Specifically, the disclosure pertains to disposable absorbent articles, preferably disposable pants, comprising elastic front and back panels, an absorbent insert, and an adjustable waist-tape.

### BACKGROUND

Disposable absorbent articles, including diapers, training pants, and incontinence products, have become essential items for managing bodily waste. These articles are designed to absorb and contain fluids, providing convenience and hygiene for wearers and caregivers. A key factor in the effectiveness and comfort of these articles is their ability to conform to the wearer's body, preventing leakage and maintaining a secure fit. Many conventional absorbent articles utilize elastic panels in the front and back regions to improve fit and comfort. These elastic components help the article stretch and contract, adapting to the wearer's movements and body contours. However, challenges remain in achieving an optimal fit for all body shapes and sizes. Variations in body proportions can lead to gaps, sagging, and subsequent leakage, particularly around the waist and leg openings.

WO 2019/005842 A1 discloses disposable absorbent articles with adjustable leg openings and secondary waist adjustment features. This document addresses the problem of leakage due to ill-fitting leg openings by incorporating adjustment straps that can be adjusted to reduce the length of the sides defining the leg openings. While this approach provides a solution for improving the fit around the legs, it does not address specific configurations for adjusting the waist circumference. The drawback of this prior art is that it does not offer a comprehensive solution for adjusting both leg openings and the waist area, potentially leading to a less secure and comfortable fit, and failing to fully address the problem of leakage around the waist area.

EP0757550B2 discloses an absorbent garment with improved fit and comfort using elastic materials in the waist region. This document focuses on enhancing the elastic properties of the waist region to provide a snug fit. While this approach improves comfort and flexibility, it does not incorporate an adjustable waist-tape that allows the user to customize the fit. The drawback of this prior art is the lack of adjustability for different body shapes, which may result in compromised fit and leakage, especially for individuals with varying waist sizes.

Therefore, the problem to be solved is to provide an absorbent article with improved adjustability and fit around the waist, addressing the limitations of existing designs by incorporating features that allow for customizable waist adjustments, thereby enhancing comfort and preventing leakage for a broader range of body shapes and sizes.

### SUMMARY

An aspect of the disclosure relates to a disposable absorbent article, preferably a disposable pant, comprises a substantially transversely extending front panel; a substantially transversely extending back panel; wherein the front and/or back panels are elastic and comprise an inner nonwoven layer, an outer nonwoven layer, and an elastic material sandwiched therebetween; and a substantially longitudinally extending absorbent insert joined to each of said front and back panels, said absorbent insert comprising front and back transversal edges and left and right longitudinal edges connecting the front and back transversal edges to form a perimeter thereof; wherein the absorbent insert comprises a substantially liquid permeable topsheet, a substantially liquid impermeable backsheet, and an absorbent core sandwiched therebetween, characterized in that said absorbent article further comprises at least a first adjustable waist-tape attached to the absorbent article in at least a first permanent attachment zone, wherein said first adjustable waist-tape comprises at least a first releasable attachment zone that releasably couples with the absorbent article such that the position at which the first releasable attachment zone is attached to the absorbent article can be adjusted to reduce the waist circumference of said absorbent article. This configuration may provide enhanced fit and leakage prevention by allowing adjustment of the waist circumference.

According to a preferred embodiment of the disclosure, the elastic material is selected from the group consisting of an elastic film, an elastic nonwoven, a plurality of elastic strands and any combination thereof. This may enhance the elasticity and conformability of the front and back panels.

According to a preferred embodiment of the disclosure, the front and back panels are joined together to define a pair of side seams. This may provide a secure and comfortable pant-like structure.

According to a preferred embodiment of the disclosure, said absorbent article is divided into a first longitudinal half and a second longitudinal half by a longitudinal center axis and wherein said at least first permanent attachment zone is permanently attached to the front or back panel in said first or second longitudinal half and said at least first releasable attachment zone is releasably attached in said other longitudinal half of said front or back panel comprising the at least first permanent attachment zone. This may allow for effective waist adjustment by ensuring the releasable zone is on the opposite side from the permanent attachment.

According to a preferred embodiment of the disclosure, adjusting the at least first releasable attachment zone extends the at least first adjustable waist-tape to the other of said front and back panels, across at least one of said pair of side seams. This configuration may allow for a secure fit and prevents the waist-tape from interfering with the side seams.

According to a preferred embodiment of the disclosure, said absorbent article is divided into a first longitudinal half and a second longitudinal half by a longitudinal center axis and wherein said at least first permanent attachment zone is permanently attached to the front or back panel along said longitudinal center axis and said at least first releasable attachment zone is releasably attached in said first or second longitudinal half of said front or back panel. This may provide a balanced and symmetrical adjustment around the waist.

According to a preferred embodiment of the disclosure, wherein said first adjustable waist-tape comprises at least a second releasable attachment zone and wherein said first releasable attachment zone of said at least first waist-tape is releasably attached to the front or back panel in one of said first or second longitudinal half and said second releasable attachment zone is releasably attached in the other of said first or second longitudinal half of said front or back panel. The addition of a second releasable attachment zone may allow a broader range of adjustability.

According to a preferred embodiment of the disclosure, adjusting the at least first releasable attachment zone extends the at least first adjustable waist-tape to the other of said back and front panels, across at least one of said pair of side seams and/or wherein adjusting the at least second releasable attachment zone extends the at least first adjustable waist-tape to the other of said back and front panels, across at least the other of said pair of side seams. This may ensure the waist-tape can effectively tighten the fit even with side seams present.

According to a preferred embodiment of the disclosure, at least a portion of said first adjustable waist-tape comprises an elastic material selected from the group consisting of elastic film, elastic fibers, elastic strands, elastic nonwovens, and combinations thereof. This may allow the waist-tape to maintain tension and conform to the wearer's body.

According to a preferred embodiment of the disclosure, the releasable attachment zone(s) are configured to engage with the outer nonwoven layer of the front or back panel. This may provide a soft and skin-friendly attachment surface.

According to a preferred embodiment of the disclosure, one or more of the releasable attachment zone(s) comprise a Velcro or hook material. This may ensure a secure and reliable attachment.

According to a preferred embodiment of the disclosure, one or more of the releasable attachment zone(s) comprise a nonwoven substrate including touch fasteners formed thereon. This may provide a gentle and effective fastening mechanism.

According to a preferred embodiment of the disclosure, the at least first adjustable waist-tape comprises an upper edge and said disposable absorbent article comprises a waist edge, wherein the distance between the waist edge and said upper edge is less than 50% of the side seam length. This may optimize the placement and adjustability of the waist-tape relative to the waist edge.

According to a preferred embodiment of the disclosure, the at least first adjustable waist-tape comprises at least a first temporary sealing zone wherein said first adjustable waist-tape is temporarily attached to the front or back panel. This may secure the waist-tape in place during manufacturing and initial use.

According to a preferred embodiment of the disclosure, said at least first temporary sealing zone is positioned between said first permanent attachment zone and said first releasable attachment zone or between said first permanent attachment zone and said second releasable attachment zone. This may ensure that the temporary seal does not interfere with the primary attachment zones.

According to a preferred embodiment of the disclosure, at least a second temporary sealing zone is positioned between said first permanent attachment zone and said first releasable attachment zone or between said first permanent attachment zone and said second releasable attachment zone. This may improve the stability and positioning of the waist-tape during manufacturing and initial use.

According to a preferred embodiment of the disclosure, the at least first and/or second temporary sealing zone are formed by a technique selected from a group consisting of ultrasonic bonding, thermal bonding, adhesive bonding. This may provide a range of effective and cost-efficient sealing methods.

According to a preferred embodiment of the disclosure, a pair of leg-openings are formed by one or more crotch-leg-peripheral-edges and front and back waist-leg-peripheral-edges and wherein a distance D, extending parallel to the longitudinal axis between the front waist edge and the apex of the front waist-leg-peripheral-edge is smaller than the side seam length extending parallel to the longitudinal axis. This may ensure that the leg openings are properly shaped for comfort and leakage prevention.

According to a preferred embodiment of the disclosure, the ratio of distance D to length L is within the range of 50% to 99%, preferably within the range of 80% to 95%, most preferably within the range of 82% to 90%. This may provide specific dimensions for the leg openings to optimize fit and prevent leakage.

According to a preferred embodiment of the disclosure, said at least first adjustable waist-tape has its longest dimension extending along the transverse direction and perpendicular to the longitudinal center axis (y). This may provide a properly shaped adjustable waist-tape for comfort and leakage protection.

According to a preferred embodiment of the disclosure, said absorbent article comprises at least a second adjustable waist-tape attached to the absorbent article in one of said front or back panels. This may allow for even greater adjustability and a more customized fit.

According to a preferred embodiment of the disclosure, said at least second adjustable waist-tape and said at least first adjustable waist-tape are attached to the same front or back panel. This may simplify the manufacturing process and may provide a more integrated design.

According to a preferred embodiment of the disclosure, said at least first adjustable waist-tape is attached to one of said front or back panels and said at least second adjustable waist-tape is attached to the other of said front or back panels. This may provide a balanced and symmetrical adjustment around the waist, optimizing fit and comfort.

### BRIEF DESCRIPTION OF THE FIGURES

The disclosure can be better understood with reference to the following drawings and description. The components in the figures are not necessarily to scale, emphasis instead being placed upon illustrating various preferred embodiments of the disclosure.
Fig. 1 is a plan view of an unassembled and fully stretched article according to an embodiment of the present disclosure.
Fig. 2 is a plan view of a cross-sectional view of the absorbent insert.
Fig. 3 is a cross-sectional view of the elastic front and back panels.
Fig. 4 illustrates two possible configurations for the adjustable waist-tape.
Fig. 5 illustrates a disposable absorbent article with an alternative embodiment.
Fig. 6 illustrates a detailed top view of the adjustable waist-tape.
Fig. 7 illustrates a touch fastener.
Fig. 8 is a cross-sectional side view of an apparatus and process for forming projections which may be used as hook-type fasteners.
Fig. 9 is a top view of the leg-openings.

### DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
The expression "% by weight" (weight percent) otherwise referred to as "wt%", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, adult pants, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The "absorbent medium" or "absorbent core" or "absorbent body" or "retention portion" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition layer", "distribution layer", "acquisition and distribution layer", "ADL" or "surge management portion" can be used interchangeably to refer to a sub-layer under the top sheet of an absorbent product, which speeds up the transport and improves distribution of fluids to the absorbent core. The surge management portion comprises a nonwoven layer, such as a nonwoven wicking layer. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present disclosure. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present disclosure.

Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The term "bio-based" may refer to a component of an absorbent article that can be produced or is derived from a renewable resource, as defined herein.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

An absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

The absorbent article can comprise leg containment gaskets. Leg "containment gaskets" help prevent leakage of bodily exudates about the legs of the wearer when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Continuous" means that the described structure is a closed-loop structure. The continuous structure may be unitary, i.e., a one-piece structure, or may be made up of individual elements suitably joined together to form a closed-loop.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "basis weight" when referring to the absorbent material, in particular the SAP, of a layer, refers to the amount of the absorbent material divided by the surface area of the layer over which the absorbent material is spread out.

The term "density" or "concentration" when referring to the absorbent material, in particular the SAP, of a layer, refers to the amount of the absorbent material divided by the volume of the layer over which the absorbent material is spread out.

"Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

As used herein, the term "elastic resistance" describes an elastic force that tends to resist an applied tensile force causing a material provided therewith to tend to contract to an untensioned configuration in response to a stretching force.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "film" refers to a sheet-like material wherein the length and width of the material far exceed the thickness of the material. Typically, films have a thickness of about 0.5 mm or less.

The term "flexible" refers to materials which are compliant and which will readily conform to the general shape and contours of the wearer's body.

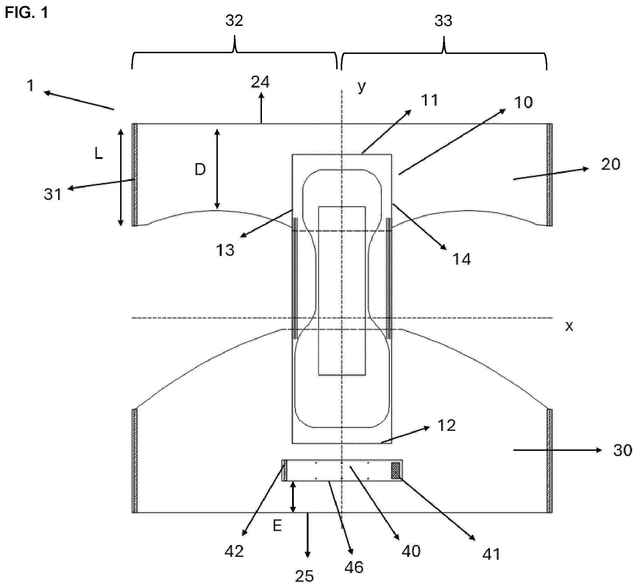

The term "fully extended state" refers to the state in which the absorbent article is laid flat, with all elastic components extended to the point where the material in which they are incorporated is maximally extended in the direction the materials are designed to elongate when in use, but without causing damage or permanent deformation to any of the article's components. For the purpose of dimensional measurements, the "fully extended state" may correspond to a 2D technical drawing, prepared by a designer for production purposes, showing a top plan view of the article in an opened-up and laid-flat state, with all elastic components extended as described above as for example illustrated by Figure 1. In case of discrepancy, the 2D technical drawing takes precedence over the physical extension of the article.

The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alphaolefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

Although the term "hook" or "hook element" is used herein when referring to a fastener element, it is understood that fastener elements are not limited to hook-like shapes and may be in the shape of mushrooms, tees, hooks, multi-lobed hooks, pins, projections or other shapes as desired. Such shapes may act as fastener elements as formed or may be post formed to make them suitable as a fastener for use in touch fastener.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" is intended to refer to the article when it is flattened into a plane or is substantially flattened into a plane and is used in contrast to when the article otherwise positioned, such as when the article is folded or shaped in or for use by a wearer.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

"Longitudinal" is a direction running parallel to the maximum linear dimension of the article.

"Loop" or "loop material" may include any structure or material that is suitable for mating with a fastener element, such as for example a hook element. The loop may be formed from a woven, nonwoven, foam, screen or mesh substrate or combinations thereof.

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" or "inelastic" refers to any material which does not fall within the definition of "elastic" above, typically by not comprising elastic materials such as elastic web materials described herein.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The term "Plant-based fibers," as that term is used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

Further, the term "projection" or "pin" may refer to an element that by itself or through a post forming operation can function as a fastener element. Further, the terms "fastener element" and "fastening elements" are used interchangeably herein.

"Pulp fluff" or "fluff pulp" refers to a material made up of cellulose fibers. The fibers can be either natural or synthetic, or a combination thereof. The material is typically lightweight and has absorbent properties.

As used herein the term "sheet" or "sheet material" refers to woven materials, nonwoven webs, polymeric films, polymeric scrim-like materials, and polymeric foam sheeting.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bidirectional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

Use of the term "substrate" includes, but is not limited to, woven or nonwoven webs, porous films, ink permeable films, paper, composite structures, or the like.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "topsheet" or "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet may be joined to portions of the backsheet, the absorbent core, the barrier leg cuffs and/or any other layers as is known to those of ordinary skill in the art. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article.

The top sheet fabrics may be composed of a substantially hydrophobic material, and the hydrophobic material may optionally be treated with a surfactant or otherwise processed to impart a desired level of wettability and hydrophilicity.

"Touch fasteners" (known commercially as Velcro^{®}, Scotchmate^{®}, Tri-Hook^{®}, etc.) were originally produced using textile technology. Two of the most common types of touch fasteners are hook and loop fasteners and mushroom and loop fasteners.

As used herein, the term "transverse" or "lateral" refers to a line, axis, or direction which lies within the plane of the absorbent article and is generally perpendicular to the longitudinal direction.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighbouring particles.

Embodiments of the articles according to the disclosure will now be described. It is understood that technical features described in one or more embodiments maybe combined with one or more other embodiments without departing from the intention of the disclosure and without generalization therefrom.

The present disclosure related to embodiments of a disposable absorbent article (1), preferably configured as a disposable pant, designed for ease of use and effective containment of bodily fluids. As described further herein, the absorbent article (1) generally comprises a substantially transversely extending front panel (20) and a substantially transversely extending back panel (30), with a substantially longitudinally extending absorbent insert (10) joined to each of said front and back panels and extending therebetween. The absorbent article (1) further comprises at least a first adjustable waist-tape (40) attached to the absorbent article (1) that can be adjusted to reduce the circumference of the absorbent article (1) at or near the waist.

In a preferred embodiment, as shown in Figure 1, the absorbent article (1) comprises a substantially transversely extending front panel (20) and a substantially transversely extending back panel (30). These panels form the main body of the absorbent article (1) and are designed to encircle the wearer's torso. The front and back panels each have longitudinally extending side edges. When the side edges are joined together, a substantially continuous waist opening is formed.

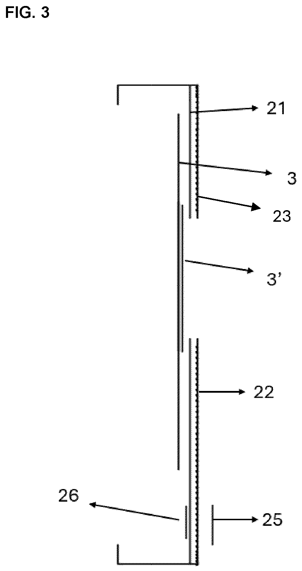

As shown in Figure 3, the front panel (20) and the back panel (30) are elastic, so that they are elastically elongatable in the transverse direction of the absorbent article (1), comprising an inner nonwoven layer (21), an outer nonwoven layer (22), and an elastic material (23) sandwiched therebetween. The elastic material (23) may extend the full transverse dimension of the front and/or back panel (20, 30) or may extend only through a portion of the respective panel(s). The elasticity of said elastic material (23) may further be modified or reduced in a portion thereof by for example cutting the elastic material (23). The inner nonwoven layer (21) is positioned closest to the wearer's skin, providing a soft and comfortable contact surface. The outer nonwoven layer (22) forms the exterior of the panels, providing structural integrity and a surface for attachment features. The elastic material (23), such as an elastic film or elastic strands, is located between the nonwoven layers, and may enable the panels to stretch and conform to the wearer's body, enhancing fit and preventing slippage. The elastic material may be joined to the inner and/or outer nonwoven by any method from the group consisting of: adhesive bonding, mechanical bonding, ultrasonic bonding, or a combination thereof.

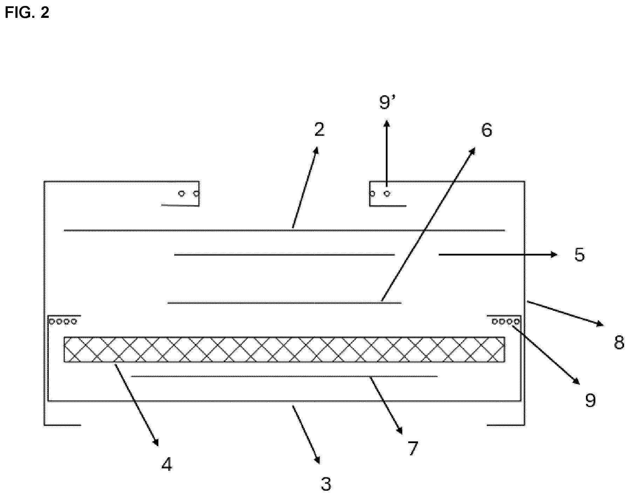

As shown in Figure 1, a substantially longitudinally extending absorbent insert (10) is joined to each of the front and back panels (20, 30). This absorbent insert (10) may be the primary component responsible for absorbing and retaining bodily fluids. The absorbent insert (10) comprises front and back transversal edges (11, 12) and left and right longitudinal edges (13, 14) connecting the front and back transversal edges (11, 12) to form a perimeter thereof. As shown in Figure 2, the absorbent insert (10) includes a liquid permeable topsheet (2), a liquid impermeable backsheet (3), an upper core wrap layer (6) and a lower core wrap layer (7) and an absorbent core (4) sandwiched therebetween. The topsheet (2) may allow fluid to quickly pass into the absorbent core (4), while the backsheet (3) may prevent fluid from leaking out of the absorbent article (1).

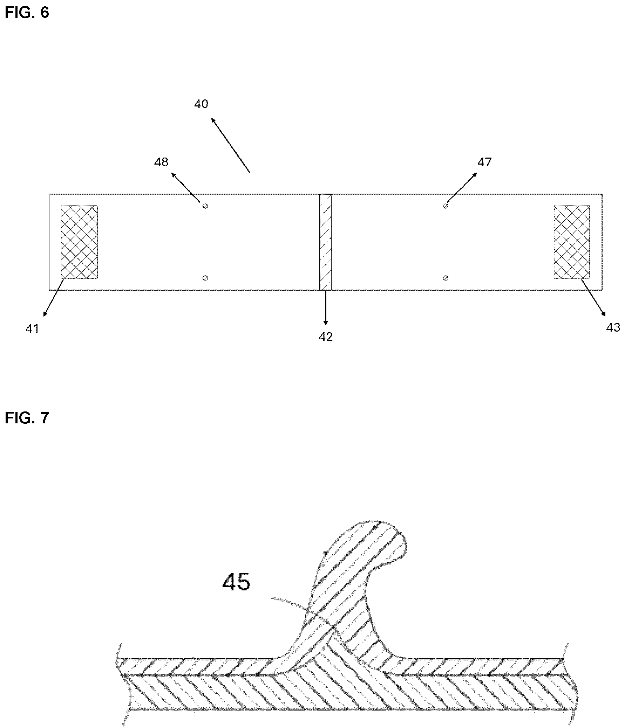

As shown in Figure 1 and Figure 6, the absorbent article (1) further comprises at least a first adjustable waist-tape (40), which includes at least a first releasable attachment zone (41) and a first permanent attachment zone (42). The first releasable attachment zone (41) may allow the wearer to adjust the tightness of the waist region, providing a customized and secure fit. The first permanent attachment zone (42) may ensure that the waist-tape is securely connected to the back panel (30), providing a reliable anchor point for the adjustment. The first permanent attachment zone (42) can be formed using various bonding techniques, such as ultrasonic bonding, thermal bonding, adhesive bonding, or stitching, or a combination thereof. This adjustable waist-tape (40) may enable the user to modify the waist circumference of the absorbent article (1) to achieve a snug and comfortable fit, reducing the risk of leakage and improving overall comfort.

In a first example, the elastic material (23) used in the front and back panels (20, 30) of said preferred embodiment, is selected from any elastic material or combination of materials that provide effective elasticity to the front and back panels (20, 30), allowing them to stretch and conform to the wearer's body. Preferably, the elastic material (23) is selected from the group consisting of an elastic film, an elastic nonwoven, a plurality of elastic strands and any combination thereof. Both elastic films and elastic strands provide effective elasticity to the front and back panels (20, 30), allowing them to stretch and conform to the wearer's body. Elastic films may offer a uniform stretch across the entire panel, providing consistent tension and support. These films can be made from various materials such as polyurethanes, styrene block copolymers, or other suitable elastomers. As shown in Figure 3, the elastic film may be sandwiched between the inner nonwoven layer (21) and the outer nonwoven layer (22), creating a smooth and comfortable surface against the skin. The elastic film may be perforated or apertured to provide improved breathability. Alternatively, a plurality of elastic strands can be used. These strands, made from materials such as natural rubber, synthetic rubber, or spandex, are arranged in a spaced-apart configuration and extending substantially in the transverse direction between the inner nonwoven layer (21) and the outer nonwoven layer (22). This configuration allows for targeted elasticity, with the strands contracting to gather the nonwoven layers and create a snug fit. Elastic strands may permit targeted elasticity, by varying various parameters including, for example, the denier, elongation, spacing of the strands. The use of elastic strands can also enhance breathability, as the spaces between the strands allow for air circulation. The choice between elastic film and elastic strands depends on the desired balance of stretch, support, and breathability for the specific application. Both options effectively contribute to the overall elasticity and conformability of the front and back panels (20, 30).

In a second example, the front and back panels (20, 30), according to any of said preferred embodiment and/or examples, are joined together to define a pair of side seams (31). These side seams (31) can create a pant-like structure, providing a secure and comfortable fit around the wearer's waist and hips. As shown in Figure 1, the side seams (31) connect the lateral edges of the front panel (20) to the lateral edges of the back panel (30), forming a closed loop or belt that encircles the wearer's lower torso, defining a waist opening. The side seams (31) can be formed using various bonding techniques, such as ultrasonic bonding, thermal bonding, adhesive bonding, or stitching, or a combination thereof. The choice of bonding method depends, at least in part, on the materials used in the front and back panels (20, 30) and the desired strength and flexibility of the seams. Ultrasonic bonding and thermal bonding can be particularly effective for nonwoven materials, creating a strong and durable bond without the need for additional adhesives. Adhesive bonding can, alternatively or additionally, also be used, providing a flexible and conformable seam. The presence of side seams (31) may ensure that the absorbent article (1) maintains its shape and position during use, preventing sagging and shifting. This pant-like configuration may enhance the overall comfort and security of the absorbent article (1), providing a reliable solution for managing bodily fluids.

In a first embodiment according to any of the preferred embodiment and/or examples, the absorbent article (1) is divided into a first longitudinal half (32) and a second longitudinal half (33) by a longitudinal center axis (y). The first adjustable waist-tape (40) is attached to the absorbent article (1) such that the first permanent attachment zone (42) is permanently attached to the front or back panel (20, 30) in the first or second longitudinal half (32, 33), and the first releasable attachment zone (41) is releasably attached in the other longitudinal half of the front or back panel (32, 33) comprising the first permanent attachment zone (42). This configuration may ensure that the adjustable waist-tape (40) can be effectively used to tighten the waist circumference, providing a customized fit. The longitudinal center axis (y) may serve as a reference point, dividing the absorbent article (1) into symmetrical halves. By positioning the permanent attachment zone (42) in one longitudinal half and the releasable attachment zone (41) in the other, the waist-tape (40) can be extended and secured to achieve the desired fit.

In a third example, adjusting the first releasable attachment zone (41), according to any of the previous embodiments and/or examples, extends the first adjustable waist-tape (40) to the other of said front and back panels (20, 30), across at least one of said pair of side seams (31). This configuration may allow the adjustable waist-tape (40) to provide a more secure and customized fit by utilizing the tension across the side seams (31). When the releasable attachment zone (41) is adjusted, the waist-tape (40) can effectively pull the front and back panels (20, 30) closer together, tightening the waist circumference. The side seams (31) preferably act as anchor points, distributing the tension and preventing localized pressure points. This configuration may be particularly beneficial for wearers who require a snug fit to prevent leakage or for those who prefer a more contoured fit.

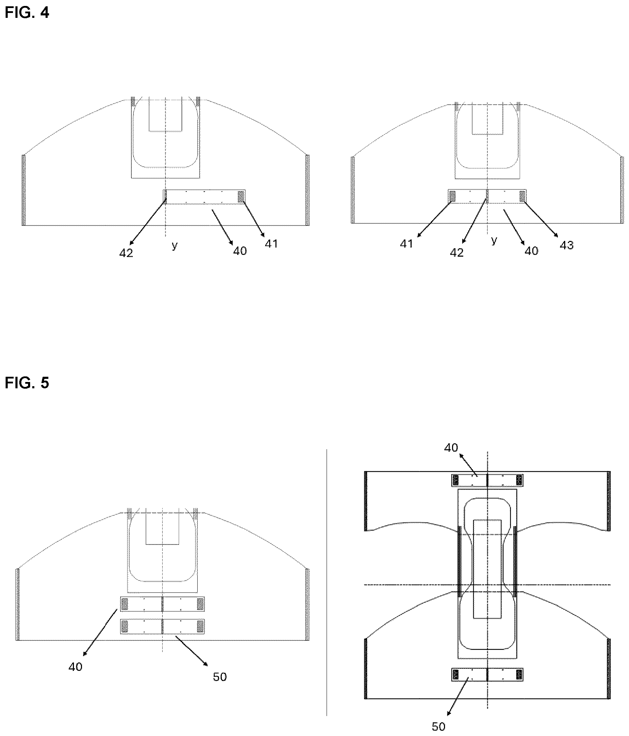

In a second embodiment, according to any of the preferred embodiment, first and/or second examples, the absorbent article (1) is divided into a first longitudinal half (32) and a second longitudinal half (33) by a longitudinal center axis (y), and the first adjustable waist-tape (40) is attached to the absorbent article (1) such that the first permanent attachment zone (42) is permanently attached to the front or back panel (20, 30) along said longitudinal center axis (y), as shown in figure 4. The first releasable attachment zone (41) is preferably releasably attached to said first or second longitudinal half (32, 33) of said front or back panel (20, 30). Preferably, the first adjustable waist-tape (40) comprises at least a second releasable attachment zone (43). The first releasable attachment zone (41) of said at least first waist-tape (40) is releasably attached to the front or back panel (20, 30) in said first or second longitudinal half (32, 33), and said second releasable attachment zone (43) is releasably attached in said other longitudinal half of said front or back panel (20, 30). As shown in Figure 4, incorporating a second releasable attachment zone (43) may provide an increased range of adjustability and customization. This configuration may allow the wearer to fine-tune the fit in 2 different directions, accommodating various body shapes and sizes and ensuring a secure and comfortable fit. The presence of two releasable attachment zones may also enhance the stability and prevents the waist-tape from shifting or folding during use. This specific placement of the permanent attachment zone (42) along the longitudinal center axis (y) may provide a balanced and symmetrical adjustment of the waist circumference. By anchoring the waist-tape (40) at the center, the tension may be distributed evenly across the front and back panels (20, 30) when the releasable attachment zone (41) is adjusted. This symmetrical adjustment may enhance comfort and minimize the risk of uneven pressure or gapping, ensuring a secure and reliable fit.

In a fourth example according to the second embodiment, adjusting the at least first releasable attachment zone (41) extends the at least first adjustable waist-tape (40) to the other of said back and front panels (20, 30), across at least one of said pair of side seams (31), and/or adjusting the at least second releasable attachment zone (43) extends the at least first adjustable waist-tape (40) to the other of said back and front panels (20, 30), across at least the other of said pair of side seams (31). This configuration may ensure that the adjustable waist-tape (40) can effectively tighten the fit, even with side seams (31) present. By extending the waist-tape (40) across one or both side seams (31), the tension may be distributed more evenly around the waist, providing a secure and comfortable fit while preventing localized pressure points.

In a fifth example according to any of the previous examples and/or embodiments, at least a portion of said first adjustable waist-tape (40) comprises an elastic material (44) selected from the group consisting of elastic film, elastic fibers, elastic strands, elastic nonwovens, and combinations thereof. This elastic material (44) may allow the waist-tape (40) to maintain tension and conform to the wearer's body, enhancing comfort and preventing sagging. The elastic material (44) can be an elastic film, providing a uniform stretch, or elastic fibers or strands, allowing for targeted elasticity and breathability. Elastic nonwovens can also be used, offering a soft and comfortable feel against the skin. The first adjustable waist-tape (40) may further comprise a first nonwoven and a second nonwoven with the elastic material (44) sandwiched therebetween. The elastic material (44) may be joined to said first and/or second nonwoven(s) by a method selected from the group consisting of: adhesive bonding, mechanical bonding, ultrasonic bonding, thermal bonding and a combination thereof.

In a sixth example according to any of the previous examples and/or embodiments, the releasable attachment zone(s) (41, 43) are configured to engage with the outer nonwoven layer (22) of the front or back panel (20, 30). This configuration may provide a soft and skin-friendly attachment surface, minimizing the risk of irritation or discomfort. The nonwoven material may also provide a suitable surface for hook-and-loop fasteners or other mechanical fasteners to securely engage.

In a seventh example according to any of the previous examples and/or embodiments, the releasable attachment zone(s) (41, 43) comprise a mechanical fastening element such as a Velcro^{®} or hook material. The hook material may comprise a strip having numerous monofilament elements, shaped like hooks. Employing Velcro or hook material in the releasable attachment zones may ensure a secure and reliable attachment, allowing the wearer to easily adjust and readjust the waist-tape as needed. These materials provide a strong and durable bond, preventing the waist-tape from unintentionally detaching during use. In an alternative embodiment, the releasable attachment zone(s) (41, 43) comprise mushroom fasteners being a strip containing numerous monofilament projections having mushroom shaped or blunted heads. In a further alternative embodiment, the hook strip(s) are formed by extruding a polymer into a web-like shape with integral projections. In the case of mushroom fasteners, the mushroom strip may be produced by extruding a polymer into a web-like shape with integral pin-like projections and post forming mushroom-like heads on the pin-like projections. Advantageously, the use of extrusion/molding technologies for the production of hook type and mushroom type touch fasteners has reduced the manufacturing cost and improved the performance and aesthetics of touch fasteners, thus allowing their use in large volume applications such as tab closures on disposable diapers. The hook material may be integrally formed with one or more other elements of the first adjustable waist-tape (40), or it may be a separate material that is joined to the first adjustable waist-tape (40).

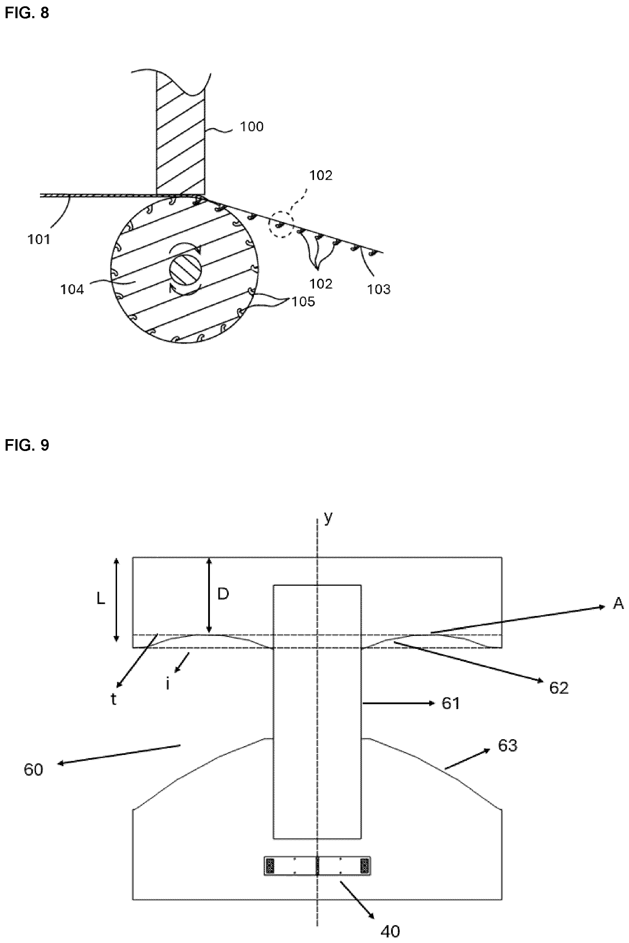

In an alternative example to said seventh example, the releasable attachment zone(s) (41, 43) comprise a nonwoven substrate including touch fasteners (45) formed thereon. Projections are preferably formed on a substrate (101) by using vibration energy. The process, as exemplarily shown in figure 8, comprises providing a mold (104) having an outer surface, providing a substrate (101) material having a surface and a device (100) as a source of vibration energy. One or both of said mold (104) and device (100) contain a plurality of cavities (105), the cavities (105) having a shape. The substrate (101) may then be positioned between the mold (104) and the device (100) and applying power to the device (100) forces a portion of the substrate (101) material to enter the cavities (105) in the mold (104) surface and forms projections (102) on a at least a portion of the surface of the substrate (101) material. The cavities (105) are shaped to form projections (102) as one or more of hooks, mushrooms, straight pins, angled pins, tapered pins, curved pins, grappling hook, multi-limbed, cross-shaped, Y-shaped and multi-lobed, each having cross-sections which are round, oval, square, rectangular, trapezoidal, solid, hollow and combinations thereof. Alternatively, the device may be pressed against the surface of the substrate material and applying power to the device forces a portion of the substrate material into the cavities in the surface of the device. Projections are generally formed on the surface of the substrate material, the projections generally conforming to the shape of the cavities. Advantageously, an effective fastening mechanism can be made without the need for high capital investment, with improved material efficiency and lower energy consumption.

In an eight example according to any of the previous examples and/or embodiments, as for example shown in figure 1, the at least first adjustable waist-tape (40) comprises an upper edge (46), which is the edge closest to the waist edge of the waist panel wherein the waist-tape is positioned and said disposable absorbent article (1) comprises a front waist edge (24) and a back waist edge (25), wherein the distance (E) between the respective waist edge (24, 25) and said upper edge (46) is less than 50% of the side seam length (L). The distances E and L are typically determined in fully extended state, the materials being maximally extended in the direction the materials are designed to elongate when in use. This particular design may optimize the placement and adjustability of the waist-tape (40) relative to the waist edge (24, 25). Limiting the distance (E) to less than 50% of the side seam length (L) may ensure that the waist-tape (40) is positioned for effective tightening and support without causing discomfort or restricting movement.

In an ninth example according to any of said previous examples and/or embodiments, as shown in figure 6, the at least first adjustable waist-tape (40) comprises at least a first temporary sealing zone (47) wherein said first adjustable waist-tape (40) is temporarily attached to the front or back panel (20, 30). The temporary sealing zone (47) secures the waist-tape (40) in place during manufacturing and initial use, preventing it from shifting or becoming misaligned. This temporary attachment may ensure that the waist-tape (40) is properly positioned for the wearer to make adjustments. Preferably, said at least first temporary sealing zone (47) is positioned between said first permanent attachment zone (42) and said first releasable attachment zone (41) or second releasable attachment zone (43). By positioning the temporary sealing zone (47) between the permanent and releasable attachment zones, the waist-tape (40) may be stabilized in a way that does not interfere with the permanent or releasable attachment zones. This arrangement may ensure that the waist-tape (40) is easy to locate and adjust when the wearer is ready to use the absorbent article (1). Preferably, at least a second temporary sealing zone (48) is positioned between said first permanent attachment zone (42) and said first releasable attachment zone (41) or second releasable attachment zone (43). The inclusion of a second temporary sealing zone (48) may further enhance the stability and positioning of the waist-tape (40) during manufacturing and initial use. This may provide additional support and prevents the waist-tape (40) from becoming distorted or misaligned before the wearer makes adjustments. The at least first and/or second temporary sealing zone (47, 48) are generally formed by a technique selected from a group consisting of ultrasonic bonding, thermal bonding, adhesive bonding. These bonding techniques offer a range of effective and cost-efficient methods for creating the temporary seal. Ultrasonic bonding and thermal bonding may provide strong and reliable bonds without the need for additional adhesives, while adhesive bonding can provide a flexible and conformable seal. The temporary sealing zones (47, 48), when present, are typically small and discreet, ensuring that they do not interfere with the primary function of the waist-tape. These zones may be formed using ultrasonic bonding, thermal bonding, or adhesive bonding, providing a secure but temporary attachment during manufacturing and initial use.

In a tenth example according to any of the previous examples and/or embodiments, a pair of leg-openings (60) are formed by one or more crotch-leg-peripheral-edges (61) and front and back waist-leg-peripheral-edges (62, 63), and wherein a distance D, extending parallel to the longitudinal axis (y) between the front waist edge (24) and the apex (A) of the front waist-leg-peripheral-edge (62) is smaller than the side seam length (L) extending parallel to the longitudinal axis (y). The apex (A) of the front waist-leg-peripheral-edge (62) is typically determined, as for example shown in figure 9, by first drawing an imaginary line (i) between the lowest point of the left side seam and the lowest point of right side seam. In a subsequent step a tangent line (t) is drawn to the front-waist-leg-peripheral-edge (62) and said tangent line runs parallel to the imaginary line (i). The apex (A) is the point where the tangent line touches the front-waist-leg-peripheral-edge (62). This may ensure that the leg openings (60) are properly shaped for comfort and leakage prevention. By controlling the distance D relative to the side seam length L, the leg openings (60) can be optimized to provide a snug and secure fit around the wearer's legs. The distances D and L are determined in fully extended state, the materials being maximally extended in the direction the materials are designed to elongate when in use. Preferably, the ratio of distance D to length L is within the range of 50% to 99%, preferably within the range of 80% to 95%, most preferably within the range of 82% to 90%. This may provide specific dimensions for the leg openings (60), further optimizing fit and preventing leakage. A ratio within this range ensures that the leg openings (60) are neither too tight nor too loose, providing a balance of comfort and security. Further preferably, said at least first adjustable waist-tape has its longest dimension extending along the transverse direction and perpendicular to the longitudinal center axis (y). This may provide a properly shaped adjustable waist-tape for comfort and leakage protection.

In a third embodiment according to any of the previous examples and/or embodiments, the absorbent article (1) comprises at least a second adjustable waist-tape (50) attached to the absorbent article in one of said front or back panels (20, 30). Including at least a second adjustable waist-tape (50) may allow for even greater adjustability and a more customized fit. With two adjustable waist-tapes, the wearer can fine-tune the tension and support around the entire waist circumference, accommodating various body shapes and sizes. The at least second adjustable waist-tape (50) may be similar in structure as the at least first adjustable waist-tape (40).

In a eleventh example according to said fourth embodiment, said at least second adjustable waist-tape (50) is attached to the absorbent article on said front or back panel (20, 30) comprising said at least first adjustable waist-tape (40). By attaching both adjustable waist-tapes (40, 50) to the same front or back panel (20, 30), the manufacturing process may be simplified, and the design is more integrated. This configuration may also provide a more intuitive and convenient adjustment mechanism for the wearer.

In a twelfth example according to any of said third embodiment and/or eleventh example, said at least first adjustable waist-tape (40) is attached to one of said front or back panels (20, 30), and said at least second adjustable waist-tape (50) is attached to the other of said front or back panels (20, 30). This configuration may provide a balanced and symmetrical adjustment around the waist, optimizing fit and comfort. By positioning one waist-tape (40) on the front panel (20) and the other waist-tape (50) on the back panel (30), the tension can be distributed evenly, preventing localized pressure points and ensuring a secure and reliable fit.

The first adjustable waist-tape (40) is preferably positioned on the back panel (30) to provide optimal adjustability and support. The width of the adjustable waist-tape (40), being the dimension of the adjustable waist-tape (40) in longitudinal direction, typically ranges from 2 cm to 10 cm, preferably from 3 cm to 9 cm, even more preferably from 4 cm to 8 cm, even more preferably from 4 cm to 7 cm, even more preferably from 4 cm to 6 cm, most preferably from 4 cm to 5 cm, providing a sufficient surface area for secure attachment and comfortable fit. If the adjustable waist-tape would be too wide it may be stiff and not conform well. The length of the adjustable waist-tape (40) is designed to extend across a substantial portion of the back panel (30), allowing for a wide range of adjustability. Advantageously, providing the tape in the back helps to reduce gapping and results in localized fit control.

The second adjustable waist-tape (50) is preferably positioned on the front panel (20) to provide optimal adjustability and support. The width of the adjustable waist-tape (50), being the dimension of the adjustable waist-tape (50) in longitudinal direction, typically ranges from 2 cm to 10 cm, preferably from 3 cm to 9 cm, even more preferably from 4 cm to 8 cm, even more preferably from 4 cm to 7 cm, even more preferably from 4 cm to 6 cm, most preferably from 4 cm to 5 cm, providing a sufficient surface area for secure attachment and comfortable fit. If the adjustable waist-tape would be too wide it may be stiff and not conform well. The length of the adjustable waist-tape (50) is designed to extend across a substantial portion of the front panel (20), allowing for a wide range of adjustability.

The specific dimensions and placement of the adjustable waist-tape (40, 50) components, in transverse and/or longitudinal direction, can be varied to optimize the fit and performance of the absorbent article (1) for different target populations, such as infants, children, or adults.

## Claims

1. A disposable absorbent article (1), preferably a disposable pant, comprising :
a substantially transversely extending front panel (20);
a substantially transversely extending back panel (30);
wherein the front and/or back panels (20, 30) are elastic and comprise an inner nonwoven layer (21), an outer nonwoven layer (22), and an elastic material (23) sandwiched therebetween;
and a substantially longitudinally extending absorbent insert (10) joined to each of said front and back panels (20, 30), said absorbent insert (10) comprising front and back transversal edges (11, 12) and left and right longitudinal edges (13, 14) connecting the front and back transversal edges (11, 12) to form a perimeter thereof;
wherein the absorbent insert (10) comprises a substantially liquid permeable topsheet (2), a substantially liquid impermeable backsheet (3), and an absorbent core (4) sandwiched therebetween, **characterized in that** said absorbent article (1) further comprises at least a first adjustable waist-tape (40) attached to the absorbent article (1) in at least a first permanent attachment zone (42), wherein said first adjustable waist-tape (40) comprises at least a first releasable attachment zone (41) that releasably couples with the absorbent article (1) such that the position at which the first releasable attachment zone (41) is attached to the absorbent article (1) can be adjusted to reduce the waist circumference of said absorbent article (1).

2. A disposable absorbent article (1) according to any of the previous claims, wherein the elastic material (23) is selected from the group consisting of an elastic film, an elastic nonwoven, a plurality of elastic strands and any combination thereof.

3. A disposable absorbent article (1) according to any of the preceding Claims, wherein the front and back panels (20,30) are joined together to define a pair of side seams (31).

4. A disposable absorbent article (1) according to any of Claims 1 to 3, wherein said absorbent article (1) is divided into a first longitudinal half (32) and a second longitudinal half (33) by a longitudinal center axis (y) and wherein said at least first permanent attachment zone (42) is permanently attached to the front or back panel (20, 30) in one of said first or second longitudinal half (32, 33) and said at least first releasable attachment zone (41) is releasably attached in said other longitudinal half of said front or back panel (32, 33) comprising the at least first permanent attachment zone (42).

5. A disposable absorbent article (1) according to any of the preceding Claims, wherein adjusting the at least first releasable attachment zone (41) extends the at least first adjustable waist-tape (40) to the other of said front and back panels (20, 30), across at least one of said pair of side seams (31).

6. A disposable absorbent article (1) according to any of Claims 1 to 3, wherein said absorbent article (1) is divided into a first longitudinal half (32) and a second longitudinal half (33) by a longitudinal center axis (y) and wherein said at least first permanent attachment zone (42) is permanently attached to the front or back panel (20, 30) along said longitudinal center axis (y) and said at least first releasable attachment zone (41) is releasably attached in said first or second longitudinal half (32, 33) of said front or back panel (20, 30).

7. A disposable absorbent article (1) according to claim 6, wherein said first adjustable waist-tape (40) comprises at least a second releasable attachment zone (43) and wherein said first releasable attachment zone (41) of said at least first waist-tape (40) is releasably attached to the front or back panel (20, 30) in one of said first or second longitudinal half (32, 33) and said second releasable attachment zone (43) is releasably attached in in the other of said first or second longitudinal half of said front or back panel (20, 30).

8. A disposable absorbent article () according to any of Claims 6 to 7, wherein adjusting the at least first releasable attachment zone (41) extends the at least first adjustable waist-tape (40) to the other of said back and front panels (20, 30), across at least one of said pair of side seams (31) and/or wherein adjusting the at least second releasable attachment zone (43) extends the at least first adjustable waist-tape (40) to the other of said back and front panels (20, 30), across at least the other of said pair of side seams (31).

9. A disposable absorbent article (1) according to any of the preceding claims, wherein at least a portion of said first adjustable waist-tape (40) comprises an elastic material selected from the group consisting of elastic film, elastic fibers, elastic strands, elastic nonwovens, and combinations thereof.

10. A disposable absorbent article (1) according to any of the preceding Claims, wherein the releasable attachment zone(s) (41, 43) are configured to engage with the outer nonwoven layer (22) of the front or back panel (20, 30).

11. A disposable absorbent article (1) according to any of the preceding Claims, wherein one or more of the releasable attachment zone(s) (41, 43) comprise a Velcro or hook material.

12. A disposable absorbent article (1) according to any of Claims 1 to 10, wherein one or more of the releasable attachment zone(s) (41, 43) comprise a nonwoven substrate including touch fasteners (45) formed thereon.

13. A disposable absorbent article (1) according to any of the preceding Claims, wherein the at least first adjustable waist-tape (40) comprises an upper edge (46) and said disposable absorbent article (1) comprises a waist edge (24, 25), wherein the distance (E) between the waist edge (24, 25) and said upper edge (46) is less than 50% of the side seam length (L).

14. A disposable absorbent article (1) according to any of the preceding Claims, wherein the at least first adjustable waist-tape (40) comprises at least a first temporary sealing zone (47) wherein said first adjustable waist-tape (40) is temporarily attached to the front or back panel (20, 30).

15. A disposable absorbent article (1) according to Claim 14, wherein said at least first temporary sealing zone (47) is positioned between said first permanent attachment zone (42) and said first releasable attachment zone (41), or between said first permanent attachment zone (42) and said second releasable attachment zone (43).

16. A disposable absorbent article (1) according to Claim 15, wherein at least a second temporary sealing zone (48) is positioned between said first permanent attachment zone (42) and said first releasable attachment zone (41) or between said first permanent attachment zone (42) and said second releasable attachment zone (43).

17. A disposable absorbent article (1) according to any of Claims 14 to 16, wherein the at least first and/or second temporary sealing zone (47, 48) are formed by a technique selected from a group consisting of ultrasonic bonding, thermal bonding, adhesive bonding.

18. A disposable absorbent article (1) according to any of the preceding Claims, wherein a pair of leg-openings (60) are formed by one or more crotch-leg-peripheral-edges (61) and front and back waist-leg-peripheral-edges (62, 63) and wherein a distance D, extending parallel to the longitudinal axis (y) between the front waist edge (24) and the apex (A) of the front waist-leg-peripheral-edge (62) is smaller than the side seam length (L) extending parallel to the longitudinal axis (y).

19. A disposable absorbent article (1) according to Claim 18, wherein the ratio of distance D to length L is within the range of 50% to 99%, preferably within the range of 80% to 95%, most preferably within the range of 82% to 90%.

20. A disposable absorbent article (1) according to any of the preceding Claims, wherein said at least first adjustable waist-tape (40) has its longest dimension extending along the transverse direction and perpendicular to the longitudinal center axis (y).

21. A disposable absorbent article (1) according to any of the preceding Claims, wherein said absorbent article (1) comprises at least a second adjustable waist-tape (50) attached to the absorbent article (1) in one of said front or back panels (20, 30).

22. A disposable absorbent article (1) according to Claim 20, wherein said at least second adjustable waist-tape (50) and said at least first adjustable waist-tape (40) are attached to the same front or back panel (20, 30).

23. A disposable absorbent article (1) according to Claim 20, wherein said at least first adjustable waist-tape (40) is attached to one of said front or back panels (20, 30) and said at least second adjustable waist-tape (50) is attached to the other of said front or back panels (20, 30).
